# EUROPEAN PATENT SPECIFICATION

(11) **EP 0 412 524 B1**
(45) Date of publication and mention of the grant of the patent: **31.03.1993**
(21) Application number: 90115208.2
(22) Date of filing: 08.08.1990
(51) Int. Cl.: B65D 83/28, B05B 1/34

(54) **Disposable nozzle adapter for intranasal spray containers**
Wegwerfdüsenadapter für intranasale Sprühbehälter
Adaptateur de buse jetable pour conteneurs pulvérisants intranasaux

(30) Priority: 11.08.1989 JP 208628/89; 24.10.1989 JP 276630/89
(43) Date of publication of application: 13.02.1991
(73) Proprietor: Toko Yakuhin Kogyo Kabushiki Kaisha, Osaka-shi, Osaka-fu (JP)
(72) Inventor: Kamishita, Takuzo, Takatsuki-shi, Osaka-fu (JP); Takagi, Toshiaki, Toyama-shi, Toyama-ken (JP)
(74) Representative: Selting, Günther, Dipl.-Ing.

(56) References cited:
- EP-A- 0 131 501
- AU-B- 30 384
- FR-A- 2 443 879

## Description

### BACKGROUND OF THE INVENTION

### 1. Field of the Invention

The present invention relates to a disposable nozzle adapter for intranasal spray containers and, more particularly, to a disposable spray nozzle adapter for intranasally spraying a viscous medical solution in combination with a spray container.

### 2. Description of the Prior Art

In the treatment of rhinitis, medical sprayers or spray containers have widely been used to administer medical solutions to nasal cavities. Since any medical substances are absorbed easily through nasal mucous membranes, the intranasal administration of medical substances has attracted much interest recently for the purpose of systemic treatment.

The sprayers or spray containers of the prior art generally comprise a pressurized container or a container with a manually-operated pump, and a spray nozzle fixed to the container. If such a spray container is applied for intranasal administration, especially, for collective administration of medical substances such as, for example, influenza HA vaccine, it is obliged to use the same spray container for a number of people as the container is filled with a medical solution several or several ten times the required quantity for a dose. This makes a filthy impression on the person to be intranasally administered and causes a danger of infection of diseases if any one of the group has an infectious diseases such as acquired immune deficiency syndrome (AIDS).

An easy solution for these problems is to wipe or disinfect the nozzle of the container by a disinfectant each time. However, such an operation is troublesome and remains a filthy impression unsolved.

It may be a good solution to use a removable spray nozzle in combination with a spray container, as disclosed for example, in lying-open Japanese patent No. 60-85759 (corresponding to US patent 4,801,093). This spray nozzle comprises a top-closed cylindrical external member with a central channel, and a substantially cylindrical internal member arranged in the central channel of the external member to form a passage, said external member having a spray opening formed in the top wall of the external member and communicated with the central channel through one or more grooves carved on the inner surface of the top wall and through a cavity surrounding the spray opening.

In use, the spray nozzle is fitted on a valve stem of the spray container with a manually operated pump to complete the spray unit, and a medical solution in the container is sprayed through the spray opening by operating the pump.

This spray unit provides an excellent spraying action for a medical solution with a relatively low viscosity, but it is impossible to spray viscous medical solutions in finely divided particles. For example, if the spray unit is used for intranasal administration of medical solutions having a viscosity of 500 to 3000 cps, the solution is never sprayed in finely divided particles, but is ejected linearly like a jet because of its high viscosity. Thus, if the solution is ejected when the person breathes in, the solution would be sucked into the trachea and, worst of all, into the lungs.

### SUMMARY OF THE INVENTION

It is therefore an object of the present invention to provide a disposable nozzle adapter for intranasal spray containers, which makes it possible to spray viscous medical solutions into nasal cavities in finely divided particles of 20 to 100 µm at a wide spraying angle.

Another object of the present invention is to provide a disposable nozzle adapter for intranasal spray containers, which is easy to manufacture and simple to handle.

These and other objects of the present invention are solved by providing a disposable nozzle adapter for intranasal administration of a viscous medical solution in combination with a spray container, which comprises;
a cylindrical body having at its one end a cylindrical chamber and at the other end a central bore for attachment of said spray container, said bore being communicated with said chamber through a channel;
a rod provided on its one end at the least with a small-sized portion and middle-sized portion and arranged in the chamber of said body to form at least one channel between its external surface and the inner surface of said chamber; and
a nozzle tip having a top wall and a cylindrical portion extending therefrom, said top wall being provided with a central spray opening including at the bottom surface of the top wall a tapered recess, and swirl grooves extending from said tapered recess to the inner surface of said cylindrical portion, said swirl grooves having a cross-sectional area increasing outwardly, the cross-sectional area of said swirl groove being 0.03 to 0.08 mm² at the minimum, said nozzle tip being fitted in the opening of said chamber and engaged with the middle-sized portion of said rod to form an annular channel surrounding said small-sized portion and being communicated with said grooves.

Particularly, the disposable nozzle adapter of the present invention is suitable for administration of viscous medical solutions with a viscosity of 500 to 3000 cps and, especially, those containing at least one carboxyvinyl polymer as a thickening agent and/or dispersion stabilizer and having a viscosity ranging from 500 to 3000 cps. It is preferred that the spray opening of the nozzle tip has a diameter ranging from 0.2 to 0.4 mm.

In one preferred embodiment, the adapter body is provided on its inner wall with longitudinally extending plural ribs which extend in parallel with the center axis of the body to form channels for the solution between its inner surface and the rod arranged therein.

In the present invention, the viscous medical solution ejected into the adapter is accelerated while passing through the swirl grooves, whirled in its tapered recess of the spray opening and then sprayed in finely divided particles with a diameter of 20 to 100 µm at a wide spraying angle. Thus, the disposable nozzle adapter according to the present invention makes it possible to spray the medical solution in finely divided particles even if the solution has a high viscosity ranging from 500 to 3000 cps.

Since the nozzle adapter of the present invention is removably fitted on the nozzle of the spray container, the adapter can be replaced with new one each time, thus making it possible to prevent the infection of deceases even when the intranasal spray unit is applied for collective administration of viscous medical solutions such as influenza HA vaccine.

The invention will be further apparent from the following description with reference to the accompanying drawings which show, by way of example only, one preferred embodiment thereof.

### BRIEF EXPLANATION OF THE DRAWINGS

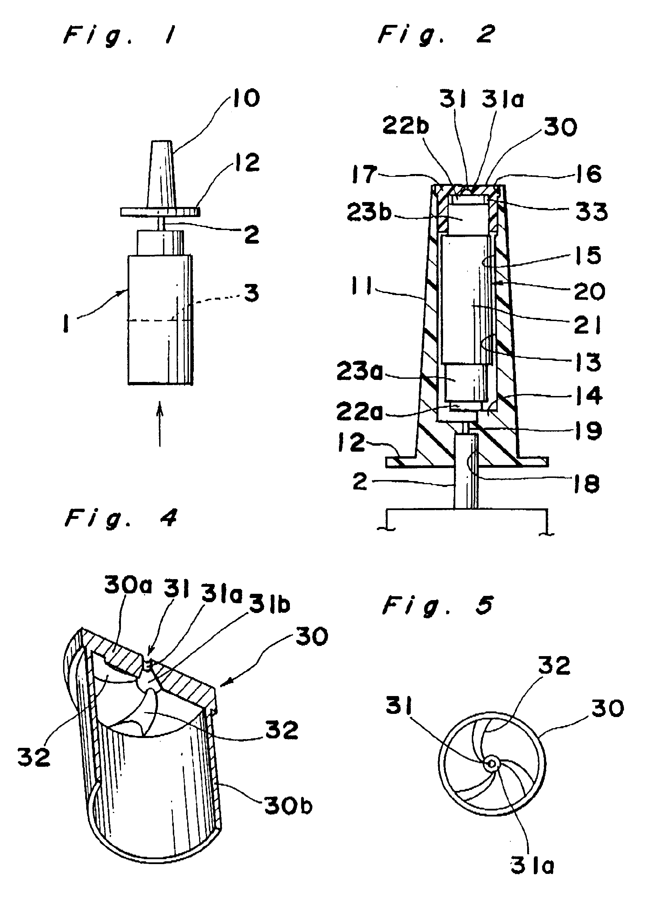

Fig. 1 is a side view of a medial spray unit with a disposable nozzle adapter embodying the present invention;
Fig. 2 is a partial section view of a medical spray unit shown in Fig. 1;
Fig. 3 is an exploded perspective view of a disposable nozzle adapter shown in Fig. 1;
Fig. 4 is an enlarged perspective section view of a nozzle tip shown in Fig. 3; and
Fig. 5 is a bottom view of a nozzle tip shown in Fig. 2.

### PREFERRED EMBODIMENTS OF THE INVENTION

Referring to the drawings, there is shown a intranasal spray unit comprising a spray container 1 with a nozzle 2, and a disposable nozzle adapter 10 removably fitted on the nozzle of the container 1. The spray container 1 is of a well-known type and has a manually-operated pumping means (not shown) to discharge a viscous medical solution 3 contained therein.

The viscous medical solution is incorporated with a carboxyvinyl polymer as a thickening agent and/or a dispersion stabilizer so that it has a viscosity ranging from 500 to 3000 cps.

The spray nozzle adapter 10 comprises an adapter body 11, a rod 20 arranged in a cylindrical chamber 13 of the body 11 and a spray nozzle tip 30 fitted in the top of the chamber 13.

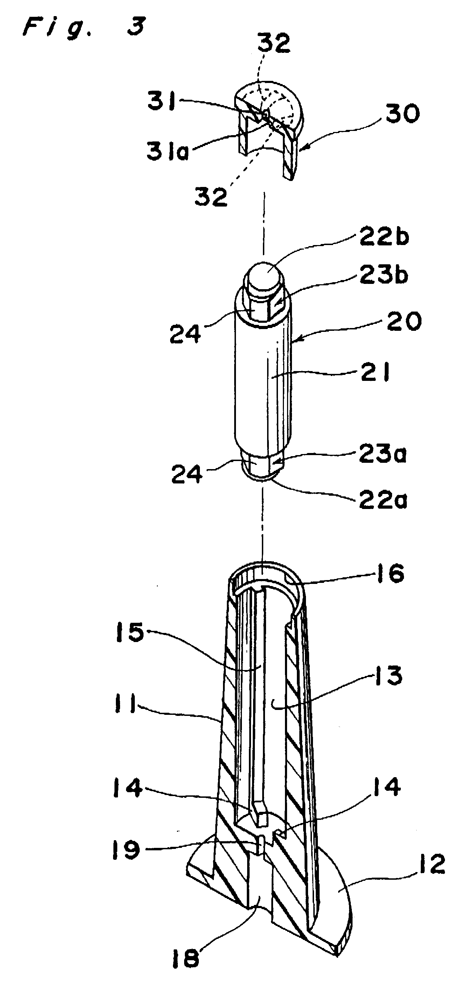

The adapter body 11 has a cylindrical shape capable of being inserted into nasal cavities, and is provided at its lower end with a flange 12. The adapter body may have any other configurations, provided that it can be inserted into nasal cavities. The cylindrical chamber 13 has, at its top portion, a widened opening 16 for attachment of the nozzle tip 30. The body 11 has a bottom provided with a central bore 18 communicated with the chamber 13 through a passage 19 formed in the bottom wall for the chamber 13. On the interior wall of the chamber 13 is provided with longitudinally extending plural ribs 15 (in this embodiment three ribs are provided) which extend in parallel with the center axis of the body and which are arranged at an angle of about 120° one another to prevent the rod 20 from shaking or displacement as well as to form channels for the solution. As best shown in in Fig. 3, the ribs 15 extends from the top end of the interior wall of the chamber 13 to the bottom where the ribs 15 are connected with projections 14, respectively.

The rod 20 is provided at its both ends with a middle-sized portion 23a, 23b and a small-sized portion 22a, 22b coaxially extending therefrom. Each middle-sized portion 23a, 23b is partially cut away to form projections 24 with a circular arc cross section as well as to form channels for the medical solution. The small-sized portion 22a is placed on the projections 14.

The rod 20 is so designed that the outside diameter of the barrel 21 is equal to or slightly smaller than that of the diameter of a circle inscribed to the projections 15, while the diameter of the small sized portion 22b is smaller than that of the nozzle tip 30 to form an annular channel surrounding the small-sized portion.

The nozzle tip 30 is a top-closed cylindrical member with a flange. The flange is fitted in the widened opening 16 of the body 11 and placed on the stepped portion 17 of the body 11. The nozzle tip 30 is provided at its top wall 30a with a spraying opening 31 composed of a straight portion 31a and a tapered recess 31b, as best shown in Fig. 4. The straight portion 31a of the spray opening 31 has a diameter ranging from 0.2 to 0.4 mm and a length of 0.2 mm. The tapered portion 31b extends downwardly from the lower end of the straight portion 31a at an opening angle of 86 to 126° and is communicated with three swirl grooves 32 carved on the bottom surface of the top wall 30a. The cylindrical portions 30b of the nozzle tip 30 is so designed that they have a diameter substantially equal to the outer diameter of the middle-sized portion 23b. Thus, the nozzle tip 30 can be fixed to the rod 20 by press-fitting its cylindrical portion 30b on the projections 24.

The swirl grooves 32 have a semicircular or semi-elliptical cross section gradually increasing outwardly. The grooves have a width of 0.15 to 0.4 mm and a depth of 0.15 to 0.25 mm at the portion where it is communicated with the tapered portion 31b. The size of the grooves is so determined that each groove has the minimum cross sectional area of 0.03 to 0.08 mm². If the cross sectional area is less than 0.03 mm², it is difficult to spray the viscous medical solution as the flow rate of the solution is considerably decreased. In addition, there is a fear that the grooves are stopped up during operation. If the cross sectional area of the groove exceeds 0.08 mm², the medical solution is not atomized into finely divided particles.

In use, the nozzle adapter 10 is held, for example, with the middle and index fingers so as to hook these fingers around the flange 12 and then inserted into nasal cavity. Then, the container 1 is pushed by the thumb to allow the container 1 to slide along its nozzle 2 toward the flange 12 of the adapter 10. The medical solution in the container 1 is forced into the nozzle adapter 10 through the passage 19, passes into the passage formed between the bottom of the chamber 13 and the rod 20, passages formed between the rod 20 and the internal surface of the body 11 and partitioned by the ribs 15, enters into the annular channel surrounding the small-sized portion 22b of the rod 20, and flows into the spraying opening through the swirl grooves. During passing though the swirl grooves, the solution is accelerated because of the decrease in the cross sectional area of the grooves, whirled in the tapered recess 31b of the spray opening, and then sprayed through the straight portion of the opening 31.

In that manner, the spray container may be used several times by replacing the nozzle adapter with new one every spraying, provided that the spray container is filled with a viscous medical solution several times the required quantity for a dose.

In fact, the medial solution containing a carboxyvinyl polymer and having a viscosity of 500 to 3000 cps was sprayed in finely divided particles with particle sizes of 20 to 100 µm.

In the above embodiment, a tapered cylindrical hollow member is used for the adapter body, but there is no restriction in shapes of the adapter body. The adapter body may take any desired shapes as occasion demands.

For example, the adapter body 11 can be modified so that it has a cylindrical portion extending from the periphery of the flange 12. Also, the adapter body 11 may have a pair of flanges extending diametrically from its lower end, instead of the annular flange 12. Further, the adapter body 11 may be provided at its barrel with two or more projections extending radially in diametrically opposed directions from each other to permit an operator to hook two fingers around the flanges as well as to allow for easy handling of the spray unit. In any cases, the size and shape of spray nozzle may be determined optionally so as to adjust a spraying angle and size or size distribution of particles sprayed.

Since the rod has small-sized portions 22a, 22b with arch-shaped projections 23a, 22b, the rod may be loaded into the body in any directions, thus making it easy to assemble the adapter. However, the rod may have one small-sized portion 22a with an arch-shaped projections 24 on one side which interacts with the nozzle tip 30.

## Claims

1. A disposable nozzle adapter for intranasal administration of a viscous medical solution in combination with a spray container, which comprises;
a cylindrical body (11) having at its one end a cylindrical chamber (13) and at the other end a central bore (18) for attachment of said spray container, said bore being communicated with said chamber through a channel (19);
a rod (20) arranged in the chamber of said body to form at least one channel between its external surface and the inner surface of said chamber; and
a nozzle tip (30) having a top wall (30a) and a cylindrical portion extending therefrom, said top wall being provided with a central spray opening (31), characterized in that said rod is provided on its one end at the least with a small-sized portion (22a, 22b) and middle-sized portion (23a, 23b) and said top wall includes at the bottom surface a tapered recess (31b), and swirl grooves (32) extending from said tapered recess to the inner surface of said cylindrical portion, said swirl grooves having a cross-sectional area increasing outwardly, the cross-sectional area of said swirl groove being 0.03 to 0.08 mm² at the minimum, said nozzle tip being fitted in the opening of said chamber and engaged with the middle-sized portion of said rod to form an annular channel surrounding said small-sized portion and being communicated with said grooves.

2. A nozzle adapter according to claim 1 wherein said viscous medical solution has a viscosity ranging from 500 to 3000 cps.

3. A nozzle adapter according to claim 1 wherein said body is provided on its inner wall with longitudinally extending plural ribs (15) which extend in parallel with the center axis of the body to form channels for the solution between said rod and its inner surface.

4. A nozzle adapter according to claim 1 wherein said viscous medical solutions contains at least one carboxyvinyl polymer and has a viscosity ranging from 500 to 3000 cps.

5. A nozzle adapter according to claim 1 wherein said spray opening of the nozzle tip has a diameter ranging from 0.2 to 0.4 mm.

## Patentansprüche

1. Wegwerfdüsenadapter zur intranasalen Verabreichung einer viskosen medizinischen Lösung in Kombination mit einem Sprühbehälter, mit:
einem zylindrischen Körper (11) mit einer zylindrischen Kammer (13) an seinem einen Ende und einer Mittelbohrung (18) zum Befestigen des Sprühbehälters an dem anderen Ende, wobei die Bohrung mit der Kammer durch einen Kanal (19) verbunden ist;
einem Stab (20), der in der Kammer des Körpers derart angeordnet ist, daß er wenigstens einen Kanal zwischen seiner Außenfläche und der Innenfläche der Rammer bildet; und
einer Düsenspitze (30) mit einer oberen Wand (30a) und einem sich davon erstreckenden zylindrischen Teil, wobei die obere Wand mit einer Mittelsprühöffnung (31) versehen ist,
**dadurch gekennzeichnet,**
daß der Stab an seinem einen Ende mit wenigstens einem kleinformatigen Teil (22a,22b) und einem mittelgroßen Teil (23a,23b) versehen ist, und die obere Wand an der Bodenfläche eine sich verjüngende Ausnehmung (31b) aufweist, und Wirbelrillen (32) sich von der sich verjüngenden Ausnehmung zu der Innenfläche des zylindrischen Teils erstrecken, wobei die Wirbelrillen einen sich nach außen vergrößernden Querschnittsbereich aufweisen, wobei der Querschnittsbereich der Wirbelrille im Minimum 0,03 bis 0,08 mm², beträgt, wobei die Düsenspitze in der Öffnung der Kammer angebracht und mit dem mittelgroßen Teil des Stabs derart in Eingriff ist, daß sie einen den kleinformatigen Teil umgebenden ringförmigen Kanal bilden, der mit den Rillen in Verbindung steht.

2. Düsenadapter nach Anspruch 1, bei dem die viskose medizinische Lösung eine Viskosität im Bereich von 500 bis 3000 cps aufweist.

3. Düsenadapter nach Anspruch 1, bei dem der Körper an seiner Innenwand mehrere, sich in Längsrichtung erstreckende Rippen (15) aufweist, die sich parallel zu der Mittelachse des Körpers derart erstrecken, daß sie zwischen dem Stab und ihrer Innenfläche Kanäle für die Lösung bilden.

4. Düsenadapter nach Anspruch 1, bei dem die viskose medizinische Lösung wenigstens ein Carboxivinylpolymer enthält und eine Viskosität im Bereich von 500 bis 3000 cps aufweist.

5. Düsenadapter nach Anspruch 1, bei dem die Sprühöffnung der Düsenspitze einen Durchmesser im Bereich von 0,2 bis 0,4 mm aufweist.

## Revendications

1. Adapteur de buse jetable pour l'administration intranasale d'une solution médicale visqueuse en combinaison avec un conteneur de produit de pulvérisation, qui comprend :
- un corps cylindrique (11) ayant à l'une de ses extrémités une chambre cylindrique (13) et à son autre extrémité un alésage central (18) pour la fixation dudit conteneur de produit de pulvérisation, ledit alésage communiquant avec ladite chambre par l'intermédiaire d'un canal (19);
- une tige (20) disposée dans la chambre dudit corps pour former un canal entre sa surface extérieure et la surface intérieure de ladite chambre; et
- une extrémité (30) de buse ayant une paroi supérieure (30a) et une partie cylindrique s'étendant à partir de celle-ci, ladite paroi supérieure présentant une ouverture centrale de pulvérisation (31), caractérisé en ce que ladite tige comporte à son extrémité au moins une partie de petite dimension (22a, 22b) et une partie de dimension moyenne (23a, 23b), ladite paroi supérieure comprend à la surface inférieure un évidement conique (31b) et des rainures de tourbillonnement (32) s'étendant entre ledit évidement conique et la surface intérieure de ladite partie cylindrique, lesdites rainures de tourbillonnement ayant une aire dans une section transversale qui croit vers l'extérieur, l'aire de la section transversale de ladite rainure de tourbillonnement étant de 0,03 ) 0,08 mm² au minimum, ladite extrémité de la buse étant montée dans l'ouverture de ladite chambre et engagée dans la partie des dimension moyenne de ladite tige pour former un canal annulaire entourant ladite partie de petite dimension et communiquant avec lesdites rainures.

2. Adaptateur de buse selon la revendication 1; dans lequel ladite solution médicale visqueuse a une viscosité comprise entre 500 et 3000 cps.

3. Adaptateur de buse selon la revendication 1, dans lequel ledit corps comporte sur sa paroi intérieure plusieurs nervures s'étendant longitudinalement (15) qui s'étendent parallèlement à l'axe du corps pour former des canaux pour la solution entre ladite tige et sa surface intérieure.

4. Adaptateur de buse selon la revendication 1, dans lequel ladite solution médicale visqueuse contient au moins un polymère carboxyvinylique et présente une viscosité comprise entre 500 et 3000 cps.

5. Adaptateur de buse selon la revendication 1, dans lequel ladite ouverture de pulvérisation de l'extrémité de la buse a un diamètre compris entre 0,2 et 0,4 mm.
